# EUROPEAN PATENT SPECIFICATION

(11) **EP 1 196 211 B1**
(45) Date of publication and mention of the grant of the patent: **07.12.2005**
(21) Application number: 00941158.8
(22) Date of filing: 26.05.2000
(51) Int. Cl.: A61M 5/44

(54) **ENHANCED THAWING OF BIOPHARMACEUTICAL SOLUTIONS USING OSCILLATORY MOTION**
BESCHLEUNIGTES AUFTAUEN VON BIOPHARMAZEUTISCHEN LÖSUNGEN UNTER ANWENDUNG VON OSZILLATIONSBEWEGUNG
DECONGELATION AMELIOREE DE SOLUTIONS BIOPHARMACEUTIQUES SOUS L'EFFET D'UN MOUVEMENT OSCILLATOIRE

(30) Priority: 28.05.1999 US 136708 P; 25.05.2000 US 579846
(43) Date of publication of application: 17.04.2002
(60) Divisional application: 05300757.1
(73) Proprietor: Integrated Biosystems, Napa, CA 94558 (US)
(72) Inventor: WISNIEWSKI, Richard, San Mateo, CA 94402 (US)
(74) Representative: Vuillermoz, Bruno
(86) International application number: PCT/US2000/014783
(87) International publication number: WO 2000/072902

(56) References cited:
- WO-A-97/24152
- DE-A- 3 047 784
- US-A- 4 954 679
- US-A- 5 779 974

## Description

### BACKGROUND OF THE INVENTION:

### Field of the Invention:

This invention relates to thawing of biopharmaceutical solutions, more particularly this invention relates to enhanced thawing of biopharmaceutical solutions using linear oscillatory motion.

### Description of Related Art:

Freezing and thawing of biological materials, particularly in the form of biopharmaceutical solutions, are useful as an intermediary processing steps during production operations, or for preservation. Until now only small individual quantities of materials, or solutions, have been successfully processed. These quantities may be contained in vials, straws, test tubes, bottles, bags, pouches, trays, etc. However, the materials handling costs, possibility of small container mix-ups, and dangers of contamination during processing and handling such small quantities meant that freezing and thawing processes were disfavored unit operations. Processing larger individual quantities would reduce or eliminate the handling or contamination problems, but such processing has met with very limited success.

A significant contributor to the final product quality is the thawing step. However, in conventional techniques, the thawing time is undesirably long. In fact, lengthy thawing times, combined with layering and cryoconcentration effects during freezing, leads to significant product degradation. While in small individual quantities the thawing step could be performed relatively rapidly in a controlled way, the thawing of large individual quantities was plagued by extensive thawing times and product degradation.

Document WO 97/24152 discloses an elaborated electrical apparatus for quick thawing of a small volume of frozen liquid placed in a syringe. This syringe is moved vertically in the air.

Document DE-A-3 047 784 discloses a system for a quicker thawing of a biosolution contained in a plastic bag. It is moved elliptically around on a base plate by a driver driving a rod.

Accordingly, what is needed is a method for thawing biopharmaceutical materials, particularly biopharmaceutical solutions, in large quantities, with very good final product quality.

### SUMMARY OF THE INVENTION:

In an aspect, the invention relates to methods for thawing frozen biopharmaceutical solutions comprising providing a container that contains a biopharmaceutical solution, at least a portion of the biopharmaceutical being frozen, providing an oscillatory driver coupled to the biopharmaceutical solution; providing a heat flux into the biopharmaceutical solution; wherein the driver inducing the oscillatory motion moves the container from a first position to a second position on a surface, and from the second position toward the first position on the surface, the motions being linear.

In another aspect, the invention relates to devices for accelerated thawing of a biopharmaceutical solution comprising a container configured to contain the biopharmaceutical solution, wherein at least a portion of the biopharmaceutical solution is frozen; a heating element, coupled to the container, that provides heat flux into the container; and an oscillatory driver capable of being coupled to the biopharmaceutical solution, configured to linearly move the container from a first position to a second position on the surface and from the second position linearly toward the first position on the surface.

### BRIEF DESCRIPTION OF THE DRAWINGS:

FIG. 1 shows a side view of a container according to the invention.
FIG. 2 shows a cross-sectional view of a device according to the invention.
FIGS. 3A-B shows an elevation of a device according to the invention, together with a representation of the oscillatory motion of the device.
FIGS. 4A-L show cross-sections of various containers according to the invention.
FIGS.5A-P show overhead elevations of various containers according to the invention.
FIGS. 6A-H show overhead elevations of various containers according to the invention.
FIGS. 7A-L show overhead elevations of various containers according to the invention.

### DETAILED DESCRIPTION OF THE INVENTION:

The inventor has unexpectedly discovered that inducing linear back and forth oscillatory motion on a surface of a biopharmaceutical solution, at least a portion of which is frozen, accelerates thawing of the biopharmaceutical.

Thawing rates may be accelerated by generation of movement of partially-thawed solid-liquid mixture comprising a biopharmaceutical solution against heat transfer surfaces in a container, preferably a container configured to contain, freeze, and/or thaw a biopharmaceutical solution. This movement may be generated wherein a liquid is moving against the heat transfer surfaces and a solid in the liquid is moving against the liquid and against the heat transfer surfaces. The patterns of liquid and solid movement may or may not be similar (the floating solid mass dynamics inside the vessel may or may not be similar to the liquid mass dynamics). The dynamic movements of liquid and solid versus the container and its internal structures may turbulize the liquid phase, affect the boundary layer at the heat transfer surfaces and at the melting solid surface, and mix the liquid (important for liquid phase homogeneity). As a result, the heat transfer between the heated surfaces and liquid and solid phases of the biopharmaceutical solution is significantly enhanced. increased heat transfer rate leads to very rapid thawing. Rapid thawing reduces or eliminates product degradation present in conventional, slow thawing, processes.

A structure useful in the practice of this invention is that of L. Quan et al., Effects of Vibration on Ice Contact Melting Within Rectangular Enclosures, Transactions of the ASME 120:518-520 (May 1998). However, the inventor notes that the Quan document does not disclose use of vibration in the thawing of frozen biopharmaceutical solutions; but only pure ice, which crystallizes and melts differently than complex aqueous solutions, such as biopharmaceutical solutions. Thus the Quan document neither teaches nor suggests the present invention.

An intensive relative motion of melting solid and liquid phases is of interest. Therefore, the dynamics of those two phases is of interest. An intensive mutual motion of these two phases may further enhance the thawing rate by additional surface melting via ablation. For example, this may be accomplished by mutual movement of the vessel and the internal heat transfer surfaces or via movement of the vessel with the internal heat transfer surfaces attached to the vessel. Such an attachment may be rigid (permanent fixing), or elastic (via springs, torsion bars, cables, etc.). According to the invention, the container is driven and linearly moves, while internal structures, such as internal heat transfer structures, can vibrate or oscillate according to their natural frequency. Thus, the forced linear movement of the container is combined with the inner heat transfer surfaces dynamics and the dynamic and dampening characteristics of the liquid and solid free phases of the biopharmaceutical solution in the container. In a preferred embodiment, the inner structure(s) vibrates at natural frequency imposing micromotion on the liquid phase. This micromotion is transferred into the solid product through the liquid. A macromotion is imposed upon the container, resulting in mutual movement of liquid and solid phases and their relative movement versus the container walls and internal structures. In such a way the container and the internal heat transfer surfaces move differently (a relative movement is induced) and the liquid and solid phases move in another pattern. This generates high mixing and turbulence rates in the liquid phase and a very high heat transfer coefficient at the heat transfer surfaces and the solid-liquid interface of the melting solid. As a result, the thawing rate is very high.

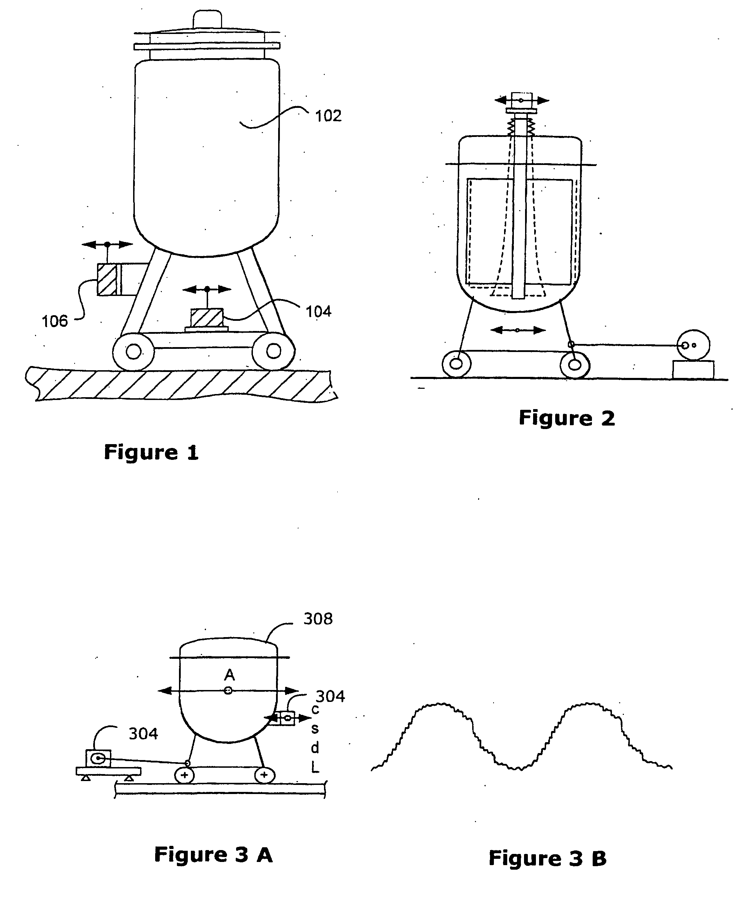

A system according to the invention is shown in FIG. 1, which shows wheeled container 102 and drivers 104 and 106 inducing oscillatory motion of a biopharmaceutical solution within the wheeled container.

A basic harmonic motion (for instance, a sinusoidal amplitude/frequency pattern) may be used to force the movement of the inventive device or to practice the inventive method. Other types of oscillatory movements also may be applied to take full advantage of the dynamics of the container, its internal structures, and the solid and liquid phases contained by it. The oscillatory motion according to the invention may be disharmonic. For example, the oscillatory motion may be in a form of asymmetrical amplitude/frequency curves (induced, for example, by preprogrammed movement of a driver coupled to the container). The movement pattern may include asymmetrical sinusoidal waves, sinusoidal waves with cut off tops/bottoms or cut sideways. Square or triangular waves (symmetrical or asymmetrical sawtooth wave shape) movement patterns may also be applied. Combinations and variations of the above are also contemplated by the invention. These patterns may have variations in amplitude and frequency, and various combinations of waveforms, wherein each waveform may have differing amplitude and frequency, may be used. For example, a small sine wave may be superimposed over a large sine wave (same with other oscillatory shapes beyond the sine waves - for example, involving shapes as described above). In addition to amplitude and frequency acceleration and deceleration patterns not only the container with the internal structures but also the resulting dynamics (acceleration and deceleration) of the liquid and solid phases are considered. Movement periodicities may be selected given consideration of the container and liquid and solid phase dynamics. Aperiodic oscillatory motions may also be used. For example, a step movement back and forth with varying periods of no motion in between the step movements may be used. Orbital oscillatory motions may also be used in the practice of this invention.

An example of oscillatory motion according to the invention may be seen in FIG. 2 illustrates a container rolling back and forth in a linear motion or surface, using a motor driving a cam.

The phenomena in the liquid-solid mixture caused by small amplitude, high frequency motion (termed "micromotion") and large amplitude, low frequency motion (termed "macromotion") may be very different. Micromotion frequencies may preferably be greater than 200 Hz, with micromotion amplitudes preferably being less than 3.0 mm. Macromotion frequencies may preferably be 200 Hz or less, with macromotion amplitudes preferably being 3.0 mm or greater. During micromotion, there are small amplitude, high frequency waves in liquid phase reaching the melting surface with the frozen mass remaining steady, while, in macromotion, there is a significant mutual macro movement between the solid and liquid phases.

The difference in physical phenomenona causes difference in thawing mechanisms. For example, macromotion thawing may result in improved product yield versus micromotion thawings. Micromotion thawing might induce vibration of ice crystals in the frozen product mass in relation to the softening glassy states around the ice crystals. Since the biopharmaceutical or pharmaceutical material may be embedded in those glassy states, the product may be exposed to effects of mechanical and hydrodynamic shear forces. These shear forces can be minimized by selection of the biopharmaceutical solution solutes and their concentration. Low amplitudes and high frequencies can make the ice crystals to vibrate stationary positions, thus minimizing the effect of ice crystal movement on the softened glassy state with embedded product. The waves will be then transferred through the softened glass states with the corresponding dampening effect of such glassy states. The ice crystals will behave as almost elastic bodies submerged into the soft, rubbery or viscous substance. The dampening effect reduces the penetration of waves deeply into the solid substance, although deeper in the solid where the temperatures are lower, the glassy states are rigid and can transfer waves as solids. Thus after the dampening of waves near the surface, the waves may travel through the solid rigid body. Such microscopic motion may disturb the ice matrix at the solid product surface and rapidly release ice crystals from the softened glassy states containing product. This may lead to a significant increase in the thawing rate.

This is a process significantly different than thawing ice only, as in Quan, where there are no softened glassy states among the ice crystals near the frozen material surface. In pure ice the crystals melt because of the liquid phase movement at the solid ice surface. Here, the movement of ice crystals and the softened glassy state (with the biopharmaceutical or pharmaceutical material in it) can be carried deeply into the material (depending on the glass transition temperature of the glassk/amorphous state; for example, if sucrose has the glass transition temperature of -32 C and the center of the frozen product is at about - 45 C with the temperature gradient towards the frozen mass surface being 45 C/ 100 mm then the depth of such softened material (mixture of the ice crystals and softened glassy states based on sucrose) will be approximately 71 mm e.g. majority of the solid material. At such large proportion of softened material between the ice crystals, the block of solid material may rapidly disintegrate if additional motion (macromotion) is imposed. This process is unique for this application and completely different than in the case of the ice thawing where the direct wave effect on the ice crystals at surface leading to some mechanical movement of ice crystal might only reach to about one crystal diameter in depth.

Macromotion as described does not involve significant deformation of the softened glassy states near the solid product surface (and not at all deeper inside the product) and therefore, well protects the biopharmaceutical product (such as for example, proteins) since there is an absence of mechanical or hydrodynamic shear in the softened glassy between the ice crystals. Macromotion can gently rearrange the ice crystals in the softened glassy states near the thawing solid surface and cause release and melting of ice crystals and dissolution of the glassy states. Therefore, this method is more versatile regarding the number of products, formulations used (types of formulation solutes) and concentration of formulation solutes.

A combination of micromotion and macromotion may be used in the practice of the invention. For example, an oscillatory movement may be constructed using a mechanical drive with a cam and a connecting arm with superimposed vibrations using a vibrator (electromagnetic, pneumatic, hydraulic, etc.) attached to the container, its internal structures or its frame. Such an embodiment is illustrated in FIGS. 3A-B. FIG. 3A shows container 308 being driven by driver 304 with a large amplitude motion in combination with driver 304 which induces a vibration. The resultant waveform is shown in FIG. 3B. The oscillatory motion may be continuously adjusted to take consideration of change of proportion of solid and liquid phases inside the container (the solid phase mass decreases, while the liquid phase mass increases).

For harmonic oscillatory motion, the range of amplitudes and frequency may be sufficiently broad so as to accelerate thawing compared to motionless thawing. In a preferable embodiment, the amplitude may range from about 0.015 mm to about 350 mm, and the frequency preferably from about 0.01 Hz to about 20 Hz, more preferably from about 0.01 Hz to about 155 kHz, still more preferably from about 0.1 Hz to about 1 kHz, and most preferably from about 0.4 Hz to about 40 Hz. These ranges may also cover superimposed harmonic movements. An example of such superimposed harmonic movements comprises an oscillatory motion with frequency of 0.5 Hz and the amplitude of 30 mm with superimposed motion of amplitude of 0.5 mm and frequency of 50 Hz.

Very often biopharmaceutical materials and/or solutions contain sensitive biological macromolecules or cell products. The biological macromolecule active product recovery level after the freezing-thawing cycle depends on the thawing rate. Generally, very rapid thawing of very small samples may provide higher yields of the product than slower thawing. The rapid thawing rate is typically of some significance in recovery of viable biological macromolecules or cells. In many cases, biological products have a strict upper temperature exposure limitation, meaning that the amount of heat applied for thawing may have an upper limit, with some safety margin imposed. For example, the heat transfer surface temperature may be limited to 8° C, 12° C, 18° C, 25°. C or 37° C depending on type of product. (these being examples only, other temperatures can be used between 0 C and, say 70 C). Sometimes, even lower temperatures are required as an upper limit. Such a low temperature of heat transfer surfaces imposes a limit upon the heat flux during thawing, because the bulk liquid phase temperature may remain close to 0° C during most of the thawing process. The liquid phase temperature may increase above this level (e.g. 0° C) at the end of thawing when the solid frozen mass disappears or its volume versus volume of liquid becomes relatively insignificant. Having the bulk liquid temperature close to 0° C during thawing is a significant factor in protecting product quality during the thawing time (some products should be stored in the liquid phase within a temperature ranging between 0° C and 4° C, or up to 8° C). During thawing, the temperature may be close to 0° C in bulk liquid, but in a boundary layer close to the heat transfer surface the temperature in the liquid phase would approach the temperature of that surface. During the stationary thawing there may be some natural convection in the liquid phase, causing some limited molecular exchange in the boundary layer (product molecules or cells residence time may still be substantial at the elevated temperatures). Such a natural convection may, however, not sufficiently agitate the liquid phase, therefore no homogenization is present and product stratification/layering may occur within the liquid volume (for example, higher product concentration may occur at the bottom). In the case of biological cells or cellular fragments (product is a suspension), a settling of suspended particles can occur. Inducing an oscillatory motion of the biopharmaceutical solution, particularly the liquid phase, not only homogenizes and mixes the liquid, but also turbulizes the boundary layer causing product molecules (or cells) residence time and exposure to elevated temperature in this layer to be significantly reduced.

Heat may be added to the biopharmaceutical solution in a variety of ways. Heat flux may be indirectly coupled to the biopharmaceutical solution via heating jackets applied to the container. Alternatively, heat flux may be directly coupled to the biopharmaceutical solution by use of submersion heaters or other submerged heat transfer surfaces, such as those disclosed in document US 6,196,296. Virtually any source of heat flux in which the heat flux can be controlled, and which source is suitable for use in biopharmaceutical applications, may be conventionally used in the practice of this invention.

The lowest permitted temperatures of heat transfer surfaces as mentioned above also limit the heat transfer driving force, e.g. temperature difference heat transfer wall-frozen product surface/bulk liquid temperature during thawing. The thawing rate may be increased only by an increase in the heat transfer surface area. The container/vessel designs as described in document US 6,196,296, possess such a large surface area of heat transfer surfaces. Further increase in heat transfer and resulting increase in the thawing rate may be accomplished by turbulization of the liquid phase. This may change the heat transfer pattern in the vicinity of the heat transfer surfaces from a laminar character to a turbulent one. From heat transfer principles it is known that, at the same temperature difference (limited for thawing of biological products). the heat transfer rate (heat flux) can be significantly increased by changing from laminar to turbulent patterns. The heat flux increase between the liquid phase and the heat transfer surface permits an increase of heat flux (container is well insulated, thus energy balance can be assumed) from the melting solid into the liquid phase. An increase in the heat flux melting solid-liquid means faster thawing rate, e.g. faster removal of the layers of the frozen product at the solid-liquid interface. An overall effect is a significant shortening of the thawing time. Oscillatory agitation with internal structures generates high rates of liquid phase turbulization inside the container. Liquid phase turbulization may cause more rapid release of ice crystals from the soft glassy state matrix at the solid mass surface.

In certain cases, biopharmaceutical materials and/or solutions may be shear sensitive and/or extensively foam when agitated. The oscillatory movement may be then adjusted to reduce the shear rate, and also wave motion on the liquid surface. Any internals of the container may also be configured to eliminate liquid flow over the structures - indeed the structures may contain the liquid and solid phase movements in compartments shaped to not only to enhance the heat transfer but also to reduce shear and foaming.

Biopharmaceutical solutions according to the invention comprise any conventional biopharmaceutical or pharmaceutical material. In preferable embodiments, biopharmaceutical solutions may comprise biological macromolecules such as proteins/enzymes, peptides, DNA, RNA, amino acids, nucleic acids, growth factors, coagulation factors, antibodies, and the like; biological cells or cell fragments/components, including bacteria, fungi, yeast, single cell organisms, mammalian (particularly human) cells, animal cells, plant cells, organelles, cell membranes, or pieces of tissue and the like; viral materials; organic or inorganic molecules or ions including carbohydrates, antibiotics; or cell growth media. Specific examples include blood and blood products (red and white blood cells, plasma, human serum albumin, etc.), and two or more phase emulsions comprising biological or pharmaceuticals materials. A separate domain comprises the vesicles, liposomes and similar membrane-based entities comprising biological components.

The inventive oscillatory motion also may include consideration for relative movement of liquid and the solid (melting) phases. This includes hydrodynamics of the liquid phase and dynamics/hydrodynamics of the floating piece of frozen product. For example, a fin (or fin/baffle) configuration inclined towards a major direction of the oscillatory motion may cause streamlined liquid flow along the fins and an elongated shape of the compartment exposes large side surface areas of the melting mass to this flowing liquid. Location of active heat transfer surfaces at the ends of the elongated compartment may cause formation of larger initial cavities there (more liquid contained there) and the movement of the container causes flow of liquid from cavity to cavity along the fins. In such a way the frozen mass is surrounded by highly turbulent flowing liquid phase, with a concomitant potential increase in thawing rates.

The oscillatory motion according to the invention may be induced in a number of ways, using a number of oscillatory drivers that are coupled to the biopharmaceutical solution. For example, the oscillatory motion may be induced mechanically, such as by an electric motor with a gear box and a cam (that may have various profiles/movement characteristics) with an arm. Alternatively, the oscillatory motion may be induced in a variety of ways, including but not limited to by an electromagnetic solenoid (push/pull), a spring loaded driver (one way movement forced), return by a spring (spring characteristics may vary - linear or nonlinear), a hydraulic drive (motor or cylinder), a pneumatic drive (motor, actuator or cylinder), or a magnetic driver (coupling/decoupling using natural or electro-magnets). The oscillatory drivers may be indirectly coupled to the biopharmaceutical solution through the container or another similar structure, or may be coupled directly to the biopharmaceutical solution, or a combination of indirect and direct coupling may be used.

An advantage of a electromagnetic driver is that the operations may be automated: rolling the container in - coupling it magnetically to the drive - processing - decoupling and rolling out. The electromagnetic driver might also be combined with safety features (decoupling if sensors detect someone approaching from a wrong side, etc.). Coupling arms may be rigid or flexible or contain an internal spring/elastic element to affect the movement characteristics and affect the system dynamics. A cable drive may also be applied (cable(s) pulling in both directions alternatively).

Driver characteristics may be further altered by shaping any rollers/wheels (for example, an off-center axis) or rails on which the container moves back and forth. The rails can be slightly curved sideways to add a side motion vector to the main movement or also be vertically curved parallel arches or alternatively located arches to add a vertical or/and sideways component to the major movement. Placing springs/elastic elements (linear or nonlinear) between the container and the rolling frame can further alter the movement characteristics. In a preferable embodiment, end motion elastic bumpers to cause a jolt (shock) at each end of oscillatory movement travel path are installed.

Agitation may be provided to further enhance thawing by enhancing the thermal transfer characteristics of the biopharmaceutical solution subject to oscillatory motion. For example, agitation may be provided by a moving internal heat transfer surface. Alternatively, it may be a rocker moving the solid phase, or it can be an oscillating frame/grid/grate located closely to active (heated) heat transfer surfaces. In another embodiment, agitation may be provided by an array of rods embedded into the frozen blocks close to their centers (last freezing, last melting parts). The array of those rods may then be driven/moved in oscillatory pattern, moving most, if not all, of the frozen portions together within the liquid phase which surrounds them (movement cannot start until sufficient portion of the liquid phase forms). In another embodiment, agitation may be provided in the form of a perforated plate with converging/diverging perforations causing a directional flow of liquid phase. Alternatively, instead of a perforated plate, a frame with "funnels" may be used; oscillatory frame movement would cause directional liquid flow through the funnels. Shaft agitators with paddles at the end could also be used, but the shaft movement instead of being rotational is preferably oscillatory. The paddles and part of the shaft may be embedded in the solid frozen material and move it through the liquid phase. The agitators moving the solid phase in the liquid induce not only a mutual liquid-solid product motion, but also turbulize the liquid around the moving solid and therefore, increase the heat transfer between liquid and heat transfer surfaces, thus cause accelerating thawing.

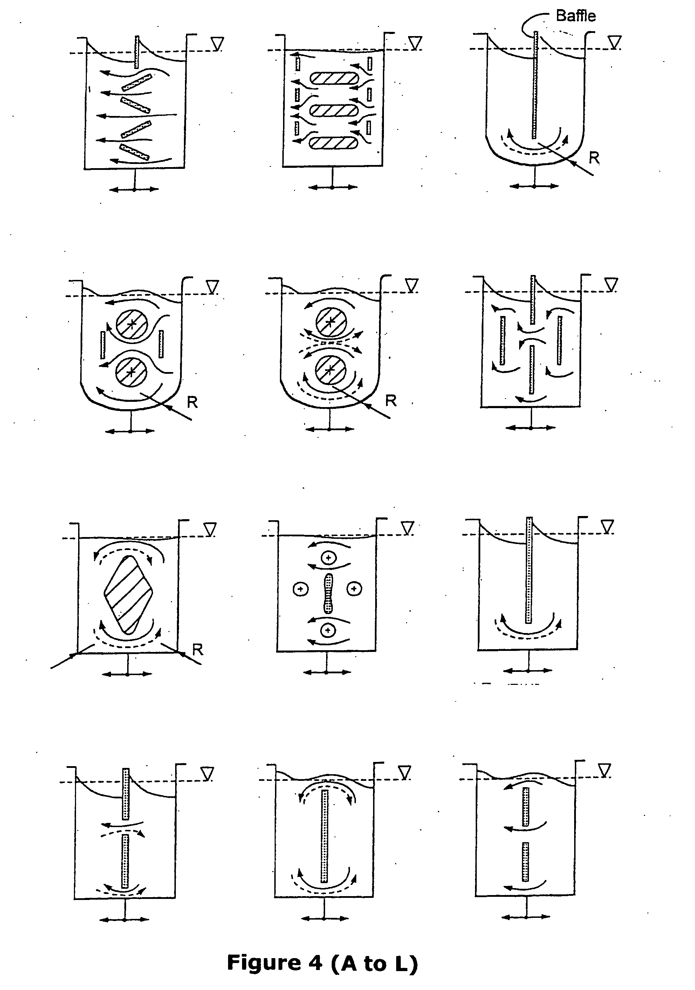

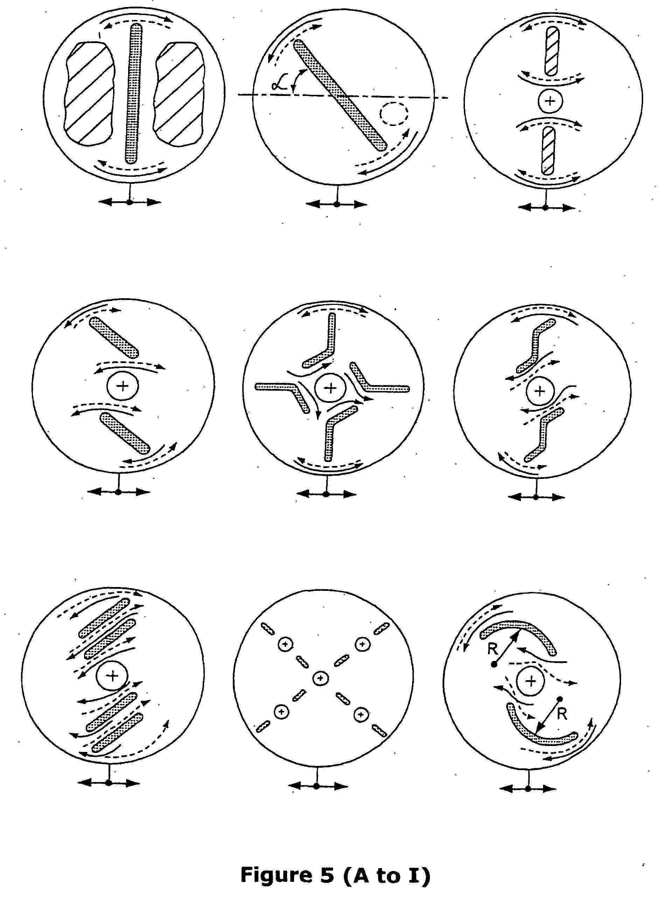

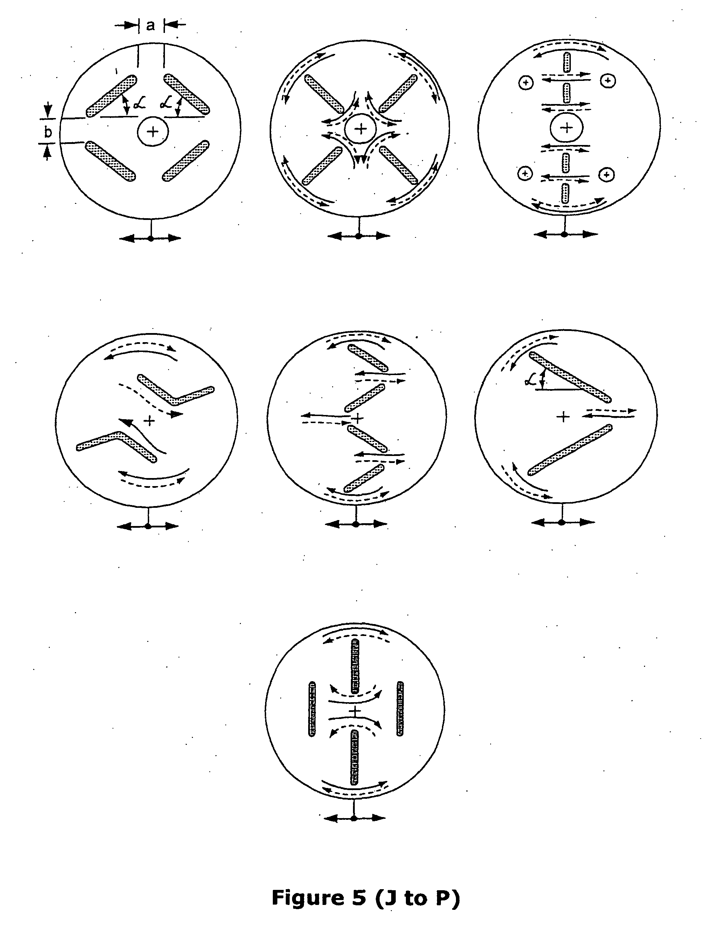

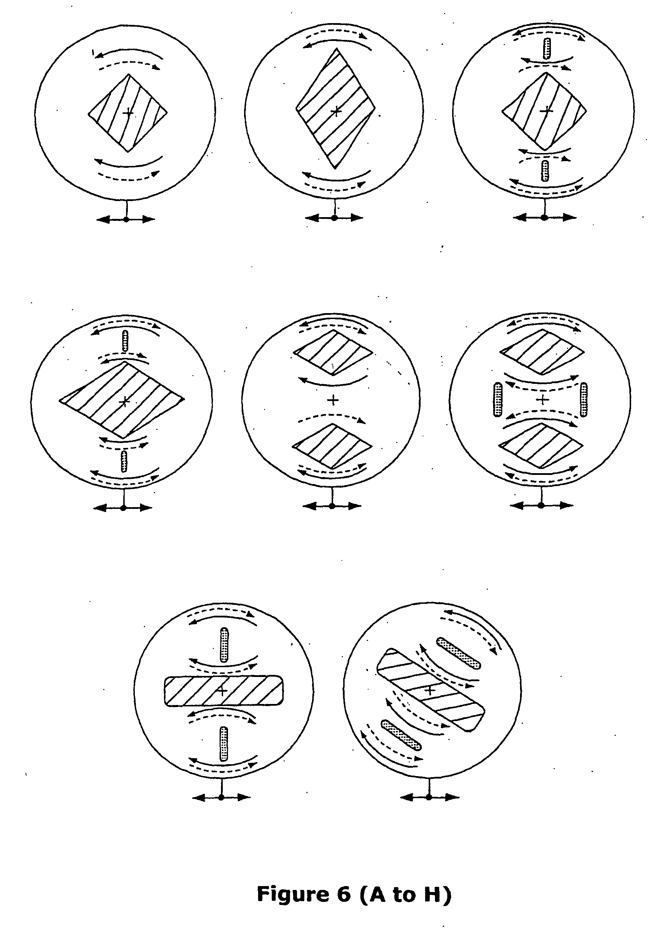

Internal structures present in the container can combine the tasks of heat transfer enhancement and intensify agitation/mixing during oscillatory motion. For example, internal heat exchangers with fins acting on the principle of thermal bridges formed during freezing, as described in document US 6,196,296, may also act as stationary mixing baffles/barriers to a wavy movement of liquid phase inside the container. Any thermal bridge gaps present will open at the beginning of the warming/thawing process permitting the liquid phase to flow around the fins between the compartments. Such a flow pattern turbulizes the liquid phase near heated surfaces and significantly increases the heat transfer and as a result the thawing rate. Cross sections of containers having such internal structures according to the invention are shown in FIGS. 4A-L. Internal structures may be actively heated (for example through use of a heat exchange fluid or an electrical resistance heater), be passively thermally conductive (such as a thermally conductive fin), or combinations of the two types of structures. FIGS. 5A-P show top views of circular cross-sections of inventive containers comprising structures according to the invention. FIGS. 6A-H show top views of circular cross-sections of inventive containers comprising structures according to the invention that include both actively heated structures and passively thermally conductive structures.

The effect is further enhanced if there are multiple active heat transfer surfaces within the container volume with a multiplicity of fins hanging between those heat transfer surfaces. The baffled structures may be baffles perpendicular to the movement of the vessel. The baffles may be placed under the angle to the main movement (to cause converging/diverging channels for the dynamically moving liquid phase with the floating (in aqueous solutions, the frozen material may float due to any density differences) solid phase. If the solid and liquid move into a converging channel of an internal structure, at one point the solid mass becomes trapped between converging structures. Its movement then stops and the relative movement of liquid phase versus that solid phase accelerates. Such an acceleration further turbulizes the boundary layer at the solid surface, thus increasing heat transfer. In addition, contact heat transfer can take place between the heat transfer structure and the thawing solid mass. Reversal of motion (second part of oscillatory movement) may release the solid, which now moves with the liquid through the diverging channel. Movements of two-phase composition through the diverging-converging channel causes additional relational movement between the solid and liquid phases causing increase in turbulence, mixing, heat transfer and resulting increase in the thawing rate.

The application of converging and diverging channels within the internal structures adds another benefit for two-phase hydrodynamics. In the converging channels the liquid may accelerate if there is an outflow at the end of the channel, while in the diverging channel the liquid phase may decelerate. If there is no outflow, the converging and diverging channels can increase the static liquid height at the channel end during converging flow, or lower the liquid level in the diverging flow. Such surface level differences in the liquid phase cause differences in static liquid pressure and increase the flow of liquid phase through the gaps around the baffles (such as any thermal bridge openings). A combination of converging and diverging channels can cause peaks and valleys on the surface of liquid phase to occur on both sides of the baffle causing a large level difference and liquid flow increase driven by this difference in static heads of liquid.

Internal structures in the container may also be configured to utilize dynamic pressure (due to the liquid mass motion) of the liquid phase (with the suspended solid phase) to propel the liquid phase through openings in the baffle or through gaps around the baffle. Multiple structures can be located "in series" for a better control of the movement of the liquid and solid phases. Heat exchangers with a radial fin configuration, such as those disclosed in document US 6,196,296, are a variation of this concept - the fins divide container volume into multiple compartments, with most of them providing baffling effect across the main direction of oscillatory movement.

The internal structures may have special flow-enhancing/directing elements shaped into them like, for example, converging-diverging nozzles. These elements may work for all situations and/or all internal structures, e.g. when the container only moves and the internal structure is fixed to the container, if the internal structure only moves and container is stationary, and when both, the container and the structure move with a relative motion against each other.

In general, the internal structures may divide the volume of the container, thus preventing formation of big liquid waves moving from side to side of the whole container. Any gaps around the internal structures, such as baffles, are beneficial since liquid moves through such gaps with relatively high velocity. The gaps may be located next to active (heated) heat transfer surfaces - particularly if the gaps serve as thermal bridges. The high velocity is turbulized and increases heat transfer rate and as a result, increases the thawing rate.

In a preferable embodiment, the internal structures may direct the liquid flow (due to dynamics caused by the oscillatory motion) at high velocity through areas in a vicinity of active (heated) heat transfer surfaces as well as agitate the bulk liquid volume. The liquid streams may be further directed towards the central part of the volume (compartment) where the floating frozen mass is located. In such a way, the liquid stream passes next to the active heat transfer surface (warms up there) and then is directed through the bulk liquid onto the melting surface passing its enthalpy to this melting surface to be converted into the latent heat of melting. Division of the container volume into compartments, and reducing large amplitude liquid phase waves may reduce splashing and foaming, thereby enhancing final product yields by reducing, for example, biological product denaturation at gas-liquid interfaces.

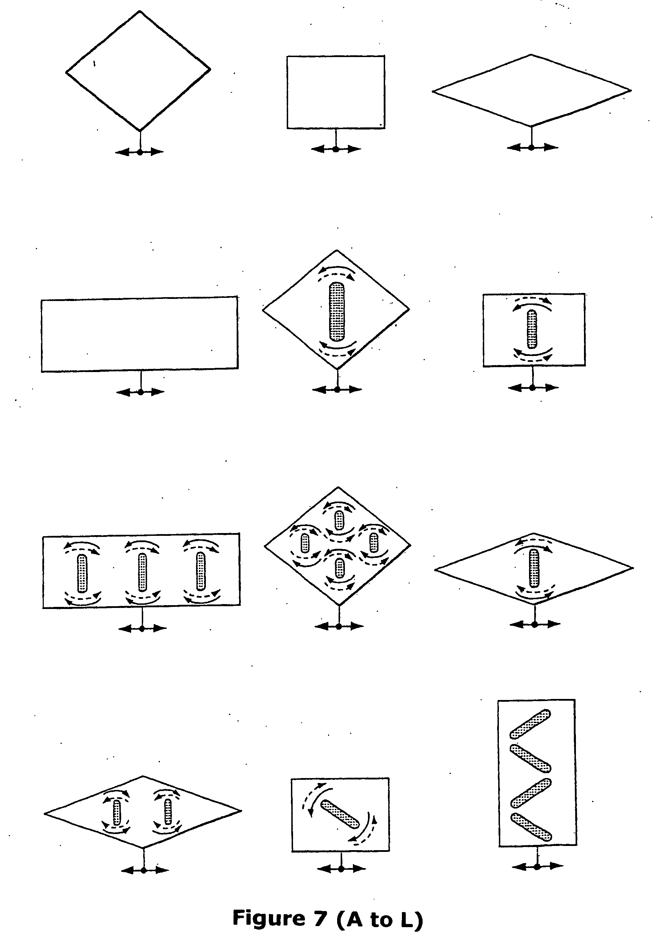

FIGS. 7A-L show top views of non-circular cross-sections of inventive containers according to the invention. FIGS. 7A-D show top views of non-circular cross-sections of inventive containers without internal structures. FIGS. 7E-L show containers according to the invention that include both actively heated structures and passively thermally conductive structures.

## Claims

1. A method for thawing a frozen biopharmaceutical solution, the method comprising:
- heating the biopharmaceutical solution held in a container, when at least a portion of the biopharmaceutical solution is frozen; and
- inducing oscillatory motion to the biopharmaceutical solution to thaw the at least a portion of the biopharmaceutical solution using an oscillatory driver configured to be coupled to the biopharmaceutical solution, wherein the driver inducing the oscillatory motion moves the container from a first position to a second position on a surface, and from the second position toward the first position on the surface,
***characterized by*** the motions being linear.

2. The method of claim 1, *wherein* the driver inducing the oscillatory motion rolls the container back and forth on a surface.

3. The method of claim 1 or 2, *wherein* the container is moved or rolled back and forth on a surface, using a motor driving a cam.

4. The method of any preceding claim, *wherein* the biopharmaceutical solution held in a container is heated using a heating element coupled to the container which contains the biopharmaceutical solution.

5. The method of any preceding claim, *wherein* the oscillatory motion of the oscillatory driver is harmonic motion.

6. The method of any preceding claim, *wherein* the oscillatory motion of the oscillatory driver is disharmonic motion.

7. The method of any preceding claim, *wherein* an amplitude of the oscillatory motion of the oscillatory driver ranges from about 0.015 mm to about 350 mm.

8. The method of any preceding claim, *wherein* a frequency of the oscillatory motion of the oscillatory driver ranges from about 0.01 Hz to about 20 Hz.

9. The method of any preceding claim, *wherein* a frequency of the oscillatory motion of the oscillatory driver ranges from about 0.1 Hz to about 1 kHz.

10. The method of any preceding claim, *wherein* a frequency of the oscillatory motion of the oscillatory driver ranges from about 0.4 Hz to about 40 Hz.

11. A device specially designed for carrying out the method of claims 1 to 10 comprising:
- a container (102, 302) configured to contain the biopharmaceutical solution, where at least a portion of the biopharmaceutical solution is frozen, said container configured to move back and forth on a surface to oscillate the biopharmaceutical solution;
- a heating element, coupled to the container, that provides heat flux into the container; and
- an oscillatory driver (104, 106, 304) configured to linearly move the container from a first position to a second position on the surface and from the second position linearly toward the first position on the surface.

12. A device according to claim 11 ***characterized in that*** the container comprises wheels or rollers.

13. A device according to claim 11 or 12 ***characterized in that*** the oscillatory driver comprises a motor driving a cam.

## Patentansprüche

1. Verfahren zum Auftauen einer gefrorenen biopharmazeutischen Lösung, wobei das Verfahren umfasst:
- Erwärmen der in einem Behälter enthaltenen biopharmazeutischen Lösung, während wenigstens ein Teil der biopharmazeutischen Lösung gefroren ist; und
- Induzieren einer oszillatorischen Bewegung in die biopharmazeutische Lösung, um wenigstens einen Teil der biopharmazeutischen Lösung aufzutauen, unter Verwendung eines Oszillations-Mitnehmers, welcher ausgebildet ist, um an die biopharmazeutische Lösung zu koppeln, wobei der Mitnehmer, welcher die oszillatorische Bewegung induziert, den Behälter auf einer Fläche von einer ersten Position in eine zweite Position und auf einer Fläche von der zweiten Position zu der ersten Position bewegt,
**dadurch gekennzeichnet, dass** die Bewegungen linear sind.

2. Verfahren nach Anspruch 1, worin der Mitnehmer, welcher die oszillatorische Bewegung induziert, den Behälter auf einer Fläche rückwärts und vorwärts rollt.

3. Verfahren nach Anspruch 1 oder 2, worin der Behälter mittels eines Motors, der eine Nocke antreibt, rückwärts und vorwärts bewegt oder gerollt wird.

4. Verfahren nach einem der vorangehenden Ansprüche, worin die in einem Behälter enthaltende biopharmazeutische Lösung mittels eines Heizelements, welches mit dem die biopharmazeutische Lösung enthaltenden Behälter verbunden ist, erwärmt wird.

5. Verfahren nach einem der vorangehenden Ansprüche, worin die oszillatorische Bewegung des Oszillations-Mitnehmers eine harmonische Bewegung ist.

6. Verfahren nach einem der vorangehenden Ansprüche, worin die oszillatorische Bewegung des Oszillations-Mitnehmers eine unharmonische Bewegung ist.

7. Verfahren nach einem der vorangehenden Ansprüche, worin die Amplitude der oszillatorischen Bewegung des Oszillations-Mitnehmers im Bereich von etwa 0,015 mm bis etwa 350 mm liegt.

8. Verfahren nach einem der vorangehenden Ansprüche, worin die Frequenz der oszillatorischen Bewegung des Oszillations-Mitnehmers im Bereich von etwa 0,01 Hz bis etwa 20 Hz liegt.

9. Verfahren nach einem der vorangehenden Ansprüche, worin die Frequenz der oszillatorischen Bewegung des Oszillations-Mitnehmers im Bereich von etwa 0,1 Hz bis etwa 1 Hz liegt.

10. Verfahren nach einem der vorangehenden Ansprüche, worin die Frequenz der oszillatorischen Bewegung des Oszillations-Mitnehmers im Bereich von etwa 0,4 Hz bis etwa 40 Hz liegt.

11. Vorrichtung, besonders ausgebildet zur Durchführung des Verfahrens nach den Ansprüchen 1 bis 10, umfassend:
- einen Behälter (102, 302), ausgebildet, eine biopharmazeutische Lösung, wo wenigstens ein Teil der biopharmazeutischen Lösung gefroren ist, aufzunehmen, der Behälter weiterhin derart ausgebildet ist, sich auf einer Fläche rückwärts und vorwärts zu bewegen, um die biopharmazeutische Lösung zu oszillieren;
- ein mit dem Behälter verbundenes Heizelement, welches einen Wärmefluss in den Behälter bewirkt; und
- einen Oszillations-Mitnehmer (104, 106, 305), derart ausgebildet, um den Behälter linear auf einer Fläche von einer ersten Position in eine zweite Position und auf einer Fläche von der zweiten Position zu der ersten Position zu bewegen.

12. Vorrichtung nach Anspruch 11, **dadurch gekennzeichnet, dass** der Behälter Räder oder Rollen aufweist.

13. Vorrichtung nach Anspruch 11 oder 12, **dadurch gekennzeichnet, dass** der Oszillations-Mitnehmer einen Motor aufweist, der eine Nocke antreibt.

## Revendications

1. Méthode pour dégeler une solution biopharmaceutique congelée comprenant :
- le chauffage de la solution biopharmaceutique contenue dans un conteneur, lorsqu'au moins une portion de la solution biopharmaceutique est congelée ; et
- l'induction d'un mouvement oscillatoire de la solution biopharmaceutique pour dégeler au moins ladite portion de la solution biopharmaceutique en utilisant un entraîneur oscillatoire configuré pour être couplé à la solution biopharmaceutique, selon lequel l'entraîneur induisant le mouvement oscillatoire déplace le conteneur d'une première position vers une seconde position sur une surface, et de la seconde position vers la première position sur la surface,
***caractérisée* en ce que** les mouvements sont linéaires.

2. Méthode selon la revendication 1, dans laquelle l'entraîneur induisant le mouvement oscillatoire roule le conteneur d'avant en arrière sur une surface.

3. Méthode selon la revendication 1 ou 2, dans laquelle le conteneur est déplacé ou roulé d'avant en arrière sur une surface, en utilisant un moteur dirigeant une came.

4. Méthode selon l'une des revendications précédentes, dans laquelle la solution biopharmaceutique contenue dans un conteneur est chauffée en utilisant un élément chauffant couplé au conteneur qui contient la solution biopharmaceutique.

5. Méthode selon l'une des revendications précédentes, dans laquelle le mouvement oscillatoire de l'entraîneur oscillatoire est un mouvement harmonique.

6. Méthode selon l'une des revendications précédentes, dans laquelle le mouvement oscillatoire de l'entraîneur oscillatoire est un mouvement disharmonique.

7. Méthode selon l'une des revendications précédentes, dans laquelle une amplitude du mouvement oscillatoire de l'entraîneur oscillatoire varie entre environ 0,015 mm et environ 350 mm.

8. Méthode selon l'une des revendications précédentes, dans laquelle une fréquence du mouvement oscillatoire de l'entraîneur oscillatoire varie entre environ 0,01 Hz et environ 20 Hz.

9. Méthode selon l'une des revendications précédentes, dans laquelle une fréquence du mouvement oscillatoire de l'entraîneur oscillatoire varie entre environ 0,1 Hz et environ 1 KHz.

10. Méthode selon l'une des revendications précédentes, dans laquelle une fréquence du mouvement oscillatoire de l'entraîneur oscillatoire varie entre environ 0,4 Hz et environ 40 Hz.

11. Dispositif adapté pour la mise en oeuvre d'une méthode selon les revendications 1 à 10 comprenant :
- un conteneur (102, 302), configuré pour contenir une solution biopharmaceutique, dans lequel au moins une portion de la solution biopharmaceutique est congelée, ledit conteneur étant configuré pour faire des allers-retours sur une surface afin d'osciller la solution biopharmaceutique ;
- un élément chauffant, couplé au conteneur, qui fournit le flux de chaleur à l'intérieur du container ; et
- un entraîneur oscillatoire (104, 106, 304) configuré pour déplacer linéairement le conteneur d'une première position vers une seconde position sur la surface et de la seconde position linéairement vers la première position sur la surface.

12. Dispositif selon la revendication 11, ***caractérisé* en ce que** le conteneur comprend des roues ou des roulettes.

13. Dispositif selon la revendication 11 ou 12, ***caractérisé* en ce que** l'entraîneur oscillatoire comprend un moteur dirigeant une came.
